# EUROPEAN PATENT APPLICATION

(11) **EP 1 118 324 A1**
(43) Date of publication of application: **25.07.2001**
(21) Application number: 01101213.5
(22) Date of filing: 19.01.2001
(51) Int. Cl.: A61K 31/19, A61P 29/00

(54) **Analgesic agent comprising a cyclobutanedicarboxylic acid derivative**

(30) Priority: 21.01.2000 JP 2000013449
(71) Applicant: Seiwa Pharmaceutical, Limited, Minato-ku, Tokyo (JP)
(72) Inventor: Nakamura, Motoyuki, Kataibaraki-shi, Ibaraki-ken (JP); Yoshizawa, Toyokichi, Kitaibaraki-shi, Ibaraki-ken (JP); Chi, Yu-Ming, Kitaibaraki-shi, Ibaraki-ken (JP); Nohara, Toshihiro, Kumamoto-shi, Kumamoto-ken (JP); Sakurada, Shinobu, Sendai-shi, Miyagi-ken (JP)
(74) Representative: Panten, Kirsten

(57) **Abstract**

An analgesic agent is provided which has analgesic action and anti-inflammatory action without significant side effects found in conventional analgesics. The analgesic agent comprises as an active ingredient a cyclobutanedicarboxylic acid derivative, containing substituted diphenyl, represented by formula (I): 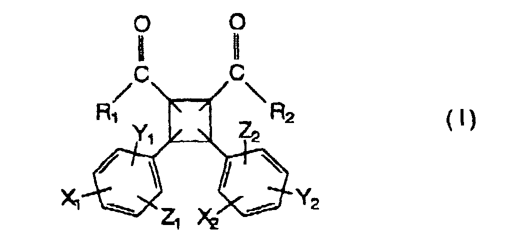 wherein X₁, X₂, Y₁, Y₂, Z₁, and Z₂, which may be the same or different, each independently represent a hydrogen atom, hydroxyl, a halogen atom, alkyl, alkoxy, or a nitrogen-containing group; and R₁ and R₂, which may be the same or different, each independently represent hydroxyl, a halogen atom, alkoxy, aryloxy, terpeneoxy, saccharide, or a nitrogen-containing group.

## Description

### BACKGROUND OF THE INVENTION

### Field of the Invention

The present invention relates to an analgesic agent, and a process for producing a derivative for use in said analgesic agent. More particularly, the present invention relates to an analgesic agent comprising as an active ingredient a cyclobutanedicarboxylic acid derivative, containing substituted diphenyl, represented by a specific chemical structural formula, and a process for producing said derivative for use in the analgesic agent.

### Background Art

In general, analgesics refer to drugs which eliminate or alleviate pain without losing consciousness. The analgesics are generally classified, for example, into: narcotic analgesics, which have been regarded as acting on the central nerve, such as morphine; non-narcotic analgesics such as aspirin and indometacin; and narcotic antagonistic analgesics which develop analgesic action through a mechanism similar to that of narcotic analgesics.

Among them, morphine is a plant-derived component, which has been begun to be used as an analgesic agent since before Christ and is still frequently used even at present, and is regarded as very important in improving the quality of life (QOL) of patients with pain.

Morphine is generally used for alleviating acute pain, such as carcinomatous pain, postoperative pain, or visceralgia. On the other hand, aspirin and non-steroidal antiphlogistic analgesics (NSAIDs), such as indometacin, are generally used for alleviating mild pain, such as arthralgia, lumbago, toothache, or contusion-derived pain. Further, they have anti-inflammatory action as well.

As generally known in the art, however, morphine has a problem that continuous use leads to an enhancement in drug dependence. Further, morphine is known to involve side effects such as constipating action and respiratory depressing action. Likewise, non-steroidal antiphlogistic analgesics typified by aspirin and indometacin inhibit the production of prostaglandin E (PGE) to develop the analgesic action, and, thus, this disadvantageously leads to side effects, for example, gastrointestinal injury, such as gastric ulcer, and renal insufficiency. Therefore, the development of novel analgesics alternative to conventional analgesics involving the above side effects, such as morphine and aspirin, has been desired in the art.

Up to now, a large number of compounds having analgesic activity have been reported. Most of them, however, are morphine-type opium alkaloids and morphine derivatives. They have high analgesic, anticonvulsant, antitussive, and stegnotic activities, but on the other hand, most of them are designated as narcotic drugs due to high dependence. For this reason, the conventional problem of side effects has not been satisfactorily solved yet.

### SUMMARY OF THE INVENTION

The present inventors have now found that specific cyclobutanedicarboxylic acid derivatives containing substituted diphenyl can exhibit excellent analgesic activity and anti-inflammatory activity and, at the same time, do not substantially involve the above-described side effects. The present invention has been made based on such finding.

Accordingly, it is an object of the present invention to provide an analgesic agent which possesses analgesic activity and anti-inflammatory activity and, at the same time, do not substantially involve side effects.

Thus, according to one aspect of the present invention, there is provided an analgesic agent comprising as an active ingredient a cyclobutanedicarboxylic acid derivative, containing substituted diphenyl, represented by formula (I): 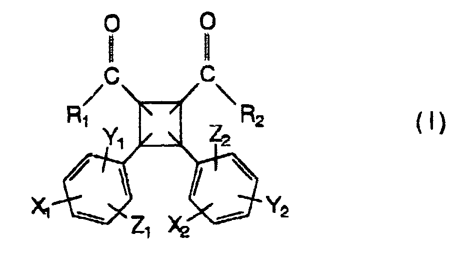 wherein X₁, X₂, Y₁, Y₂, Z₁, and Z₂, which may be the same or different, each independently represent a hydrogen atom, hydroxyl, a halogen atom, alkyl, alkoxy, or a nitrogen-containing group; and R₁ and R₂, which may be the same or different, each independently represent hydroxyl, a halogen atom, alkoxy, aryloxy, terpeneoxy, saccharide, or a nitrogen-containing group.

According to another aspect of the present invention, there is provided a process for producing a cyclobutanedicarboxylic acid derivative for an analgesic agent, said process comprising the steps of:
providing a cinnamic acid derivative represented by formula (II) 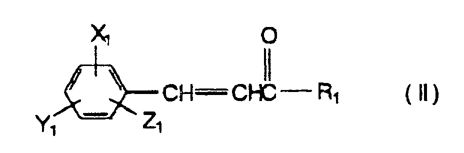 wherein X₁, Y₁, and Z₁, which may be the same or different, each independently represent a hydrogen atom, hydroxyl, a halogen atom, alkyl, alkoxy, or a nitrogen-containing group; and R₁ represents hydroxyl, a halogen atom, alkoxy, aryloxy, terpeneoxy, saccharide, or a nitrogen-containing group; and
dispersing the derivative in an organic solvent and then irradiating the dispersion with light to allow photodimerization to proceed.

The analgesic agent according to the present invention comprises as an active ingredient a cyclobutanedicarboxylic acid derivative, containing substituted diphenyl, having a structure represented by formula (I). This analgesic agent can exhibit excellent analgesic activity against pain involved in various diseases such as headache, bellyache, neuralgia, and carcinomatous pain, and, in addition, can exhibit anti-inflammatory activity. At the same time, the analgesic agent having the above constitution according to the present invention is substantially free from side effects experienced in conventional analgesic agents, for example, narcotic problems found, for example, in morphine, and gastrointestinal injury found in non-steroidal antiphlogistic analgesics such as aspirin and indometacin.

### BRIEF DESCRIPTION OF THE DRAWINGS

Fig. 1 is a diagram showing the results of an analgesic, anti-inflammatory activity test (a formalin test) in the first phase (= 0 to 10 min) upon the intraperitoneal administration of samples 1 to 9; and

Fig. 2 is a diagram showing the results of an analgesic, anti-inflammatory activity test (a formalin test) in the second phase (= 10 to 30 min) upon the intraperitoneal administration of samples 1 to 9.

### DESCRIPTION OF THE PREFERRED EMBODIMENTS

The present invention provides an analgesic agent comprising as an active ingredient a cyclobutanedicarboxylic acid derivative having a structure represented by formula (I): 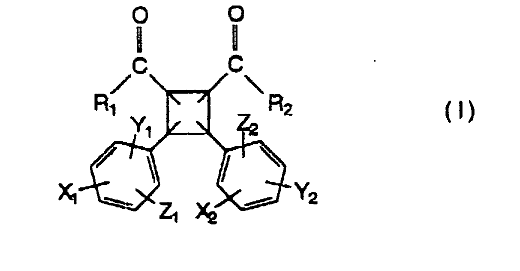 wherein X₁, X₂, Y₁, Y₂, Z₁, and Z₂, which may be the same or different, each independently represent a hydrogen atom, hydroxyl, a halogen atom, alkyl, alkoxy, or a nitrogen-containing group; and R₁ and R₂, which may be the same or different, each independently represent hydroxyl, a halogen atom, alkoxy, aryloxy, terpeneoxy, saccharide, or a nitrogen-containing group.

Here the "cyclobutanedicarboxylic acid derivative" include not only cyclobutanedicarboxylic acid compounds having a structure represented by formula (I) but also pharmaceutically acceptable salts of these compounds and, in addition, when the above compounds and salts thereof can exist as solvates (for example, hydrates), embraces such solvates. Pharmaceutically acceptable salt include salts of cyclobutanedicarboxylic acid compounds, for example, sodium and potassium salts of cyclobutanedicarboxylic acid compounds.

Comprising "as an active ingredient" means the case where a carrier suitable for a desired dosage form may be contained, as well as the case where other medicinal ingredients may be contained which may be used in combination with the derivative according to the present invention. Medicinal ingredients, which may be used in combination with the derivative according to the present invention, include, for example, conventional medicinal ingredients having analgesic activity, such as morphine, aspirin, and indometacin, and medicinal ingredients for protecting the inside of gastrointestine. Additional examples of medicinal ingredients usable in combination with the derivative according to the present invention include preservatives, binders, stabilizers, lubricants, and flavors.

The cyclobutanedicarboxylic acid derivative used in the analgesic agent according to the present invention may be one compound or a plurality of compounds selected from the group consisting of cyclobutanedicarboxylic acid derivatives represented by formula (I).

In formula (I), examples of the halogen atom in the definition of X₁, X₂, Y₁, Y₂, Z₁, and Z₂ include chlorine, fluorine, bromine, and iodine atoms. Likewise, examples of the alkyl include lower alkyl, especially C₁ to C₆ lower alkyl, for example, methyl, ethyl, propyl, and butyl. Examples of the alkoxy include those wherein the alkyl portion is lower alkyl (especially C₁ to C₆ lower alkyl), for example, methoxy and ethoxy. Further, examples of the nitrogen-containing group in the definition of X₁, X₂, Y₁, Y₂, Z₁, and Z₂ include nitro and substituted or unsubstituted amino (substituents in the case of substituted amino include, for example, lower alkyl (especially C₁ to C₆ lower alkyl).

According to a preferred embodiment of the present invention, X₁, X₂, Y₁, Y₂, Z₁, and Z₂ in formula (I) each independently represent a hydrogen atom, hydroxyl, or a halogen atom. According to a further preferred embodiment of the present invention, any one of X₁, Y₁ and Z₁ and any one of X₂, Y₂ and Z₂ both represent hydroxyl or a halogen atom while the remaining groups of X₁, X₂, Y₁, Y₂, Z₁, and Z₂ represent a hydrogen atom.

As described above, in formula (I), X₁, X₂, Y₁, Y₂, Z₁, and Z₂ may be the same or different. Therefore, a relationship is possible wherein X₁ = X₂, Y₁ = Y₂, and Z₁ = Z₂. This is one of the preferred embodiments of the present invention.

Examples of the halogen atom in the definition of R₁ and R₂ in formula (I) include chlorine, fluorine, bromine, and iodine atoms. Likewise, examples of the alkoxy in the definition of R₁ and R₂ in formula (I) include lower alkoxy, especially C₁ to C₆ lower alkyl (for example, methoxy, ethoxy, propoxy, or butoxy), or long-chain saturated or unsaturated alkoxy (especially C₇ or higher saturated or unsaturated alkoxy) (examples of substituents in this case include nitro, cyano, lower alkyl (preferably C₁₋₆ alkyl), oxy, and halogen atoms). Examples of the aryloxy include substituted or unsubstituted aryloxy having 6 to 20 carbon atoms (examples of substituents in this case include nitro, cyano, lower alkyl, oxy, and halogen atoms), for example, phenoxy, nitrophenoxy, cyanophenoxy, and naphthoxy. Examples of the terpeneoxy in the definition of R₁ and R₂ in formula (I) include monoterpeneoxy, diterpeneoxy, or triterpeneoxy. Examples of the saccharide include groups derived from monosaccharides (such as rhamnose and glucose), amino saccharides (such as glucosamine and galactosamine), or oligo saccharides (such as sucrose and trehalose). Examples of preferred saccharides include glucoside, galactoside, and rhamnoside. Examples of the nitrogen-containing group include substituted or unsubstituted amino (for example, alkylamino, phenylamino, or aralkylamino), alkaloids (for example, incarvilline), and amino acids (for example, glycine, alanine, phenylalanine).

According to a preferred embodiment of the present invention, R₁ and R₂ in formula (I) each independently represent hydroxyl, methoxy, or nitrophenoxy.

As described above, in formula (I), R₁ and R₂ may be the same or different. According to a preferred embodiment of the present invention, R₁ = R₂.

Specific examples of cyclobutanedicarboxylic acid derivatives, containing substituted diphenyl, represented by formula (I), which are contained as an active ingredient in the analgesic agent according to the present invention include: α-truxillic acid, that is, a compound represented by formula (I) wherein X₁, X₂, Y₁, Y₂, Z₁, and Z₂ = H (a hydrogen atom), and R₁ and R₂ = H; 4,4'-dihydroxy-α-truxillic acid, that is, a compound represented by formula (I) wherein any one of X₁, Y₁, and Z₁ and any one of X₂, Y₂, and Z₂ = hydroxyl, and R₁ and R₂ = H; bis(p-nitrophenyl) α-truxillate, that is, a compound represented by formula (I) wherein X₁, X₂, Y₁, Y₂, Z₁, and Z₂ = H, and R₁ and R₂ = nitrophenoxy; dimethyl α- truxillate; dimethyl 4,4'-dihydroxy-α-truxillate, that is, a compound represented by formula (I) wherein any one of X₁, Y₁, and Z₁ and any one of X₂, Y₂, and Z₂ = hydroxyl, and R₁ and R₂ = methoxy; β-truxinic acid, that is, a compound represented by formula (I) wherein X₁, X₂, Y₁, Y₂, Z₁, and Z₂ = H, and R₁ and R₂ = H; bis(p-nitrophenyl) β-truxinate; 4,4'-dichloro-β-truxinic acid; incarvillateine; and 3"-methoxyincarvillateine.

The cyclobutanedicarboxylic acid derivative, containing substituted diphenyl, represented by formula (I), contained as an active ingredient in the analgesic agent according to the present invention may be derived from natural sources or alternatively may be synthesized.

The cyclobutanedicarboxylic acid derivative used in the analgesic agent according to the present invention may be produced by any method so far as a compound represented by the above structural formula can be produced. Therefore, the derivative may be produced by providing a cyclobutane ring and introducing separately provided substituted phenyl or carboxyl into the cyclobutane ring.

According to a preferred embodiment of the present invention, the cyclobutanedicarboxylic acid derivative for use in the analgesic agent according to the present invention is produced by the following process.

Specifically, the cyclobutanedicarboxylic acid -derivative for use in the analgesic agent according to the present invention can be produced by a process comprising the steps of:
providing a cinnamic acid derivative represented by formula (II) 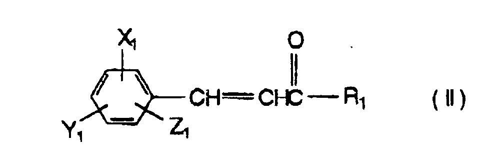 wherein X₁, Y₁, and Z₁, are as defined in formula (I) and, thus, may be the same or different and each independently represent a hydrogen atom, hydroxyl, a halogen atom, alkyl, alkoxy, or a nitrogen-containing group; and R₁ is as defined in formula (I) and, thus represents hydroxyl, a halogen atom, alkoxy, aryloxy, terpeneoxy, saccharide, or a nitrogen-containing group; and
dispersing the derivative in an organic solvent and then irradiating the dispersion with light to allow photodimerization to proceed.

Here the photodimerization refers to a reaction such that, upon photoexcitation, a molecule is bonded to the same type of another molecule on the ground state to form a 1 : 1 adduct. Light irradiation conditions are not particularly limited so far as the photodimerization can be allowed to proceed. A specific example of such conditions is that, for example, a compound dispersed in a suitable solvent in a test tube is externally irradiated with light from a high-pressure mercury lamp.

The organic solvent used in the above production process is not particularly limited so far as the solvent can disperse or dissolve therein the derivative represented by formula (II). Examples of organic solvents usable herein include hexane, tetrahydrofuran, chloroform, methylene chloride, ethyl ether, ethanol, methanol, and ethyl acetate.

The production process utilizing photodimerization according to the present invention includes the direct production of a cyclobutanedicarboxylic acid derivative for use in the analgesic agent of the present invention through the photodimerization and the production of a cyclobutanedicarboxylic acid derivative for use in the analgesic agent of the present invention in such a manner that a precursor of the contemplated derivative is produced by photodimerizaiton and is then subjected to a reaction, for example, reduction or hydrolysis, to produce the contemplated cyclobutanedicarboxylic acid derivative for use in the analgesic agent.

Embodiments of the production process of cyclobutanedicarboxylic acid derivative for use in the analgesic agent according to the present invention will be described.

For example, among the cyclobutanedicarboxylic acid derivatives according to the present invention, for example, bis(p-nitrophenyl.) β-truxinate can be produced by subjecting nitrophenyl cinnamate, as a product of a reaction of cinnamic acid chloride with nitrophenol, to photodimerization (for example, solid stage photodimerization). Here the solid stage photodimerization refers to photodimerization in a crystal state rather than a solution state.

Among the cyclobutanedicarboxylic acid derivatives according to the present invention, for example, bis(p-nitrophenyl) α-truxillate can be produced by reacting two moles of nitrophenol with one mole of truxillic acid chloride obtained by photodimerization (for example, solid stage photodimerization) of cinnamic acid in the presence of a halogenating agent such as thionyl chloride, oxalyl chloride, or phosphorus trichloride.

In the present invention, the cyclobutanedicarboxylic acid derivative containing substituted diphenyl may also be obtained from natural sources. For example, incarvillateine is contained, for example, in *Incarvillea sinensis* LAM. which is a plant belonging to the Bignoniaceae family, and thus can be extracted therefrom and purified by conventional methods.

The analgesic agent according to the present invention is provided as pharmaceutical preparations or foods. When the analgesic agent according to the present invention is used as pharmaceutical preparations, this agent is formulated, for example, into dry powders, powders, granules, tablets, sugar coated tablets, capsules, oblates, drops, and liquid preparations. In this case, the analgesic agent may contain other ingredients, for example, excipients, preservatives, binders, stabilizers, antistatic agents, lubricants, and flavors, according to contemplated dosage forms and use conditions. On the other hand, when the analgesic agent according to the present invention is used as foods, this agent can be formulated, for example, into gum, candies, jelly, tableted confectionary, and beverages.

When the analgesic agent is administered as a pharmaceutical preparation, various administration routes may be adopted. Examples thereof include oral administration, parenteral administration, inhalation, transrectal adminstration, and local administration. Among them, parenteral administration include subcutaneous injection, intravenous administration, intramuscular administration, intranasal administration, and injection. Therefore, mucosal administration or dermal administration through nose, oral cavity, hypoglottis, intestinum rectum and the like, and administration using an implant are also possible.

For example, when the analgesic agent according to the present invention is formulated into a dried powder which is then used as an injection, this powder is dissolved in distilled water, or an aqueous isotonic solution prepared using sodium chloride and a saccharide (such as glucose, mannitol, or inositol). The solution containing the active ingredient may be then administered as an injection by intravenous, intramuscular, subcutaneous, or intraorgan injection or by injection directly into a focus such as a tumor site or a portion from which a tumor has been removed, so as to provide an in vivo drug concentration useful for the target disease.

Since the ingredients of the analgesic agent do not have acute toxicity, the dose may generally be in the range of about 0.1 to 500 mg/kg of weight per administration. The analgesic agent is preferably administered one to five times per day. Preferably, the dose is properly determined in the above dose range, for example, in consideration of the age, weight, and condition of patients and the administration route.

### EXAMPLES

The following examples further illustrate the present invention, but are not intended to limit it.

Synthesis examples of analgesic agents according to the present invention will be described.

### Synthesis Example 1

Cinnamic acid (1.0 g) was provided, and then dispersed in hexane in a test tube. The dispersion was then irradiated with light for 2 days to induce photodimerization. After the completion of the reaction, the reaction product was washed several times with ether to give 900 mg of α-truxillic acid (sample 1).

The compound thus obtained was measured for physicochemical properties in terms of melting point, NMR (nuclear magnetic resonance) and the like. The results were compared with values reported in literature (A. Baracchi, S. Chimichi, F.D. Sio, D. Donati, R. Nesi, and P. Sarti-Fantoni: HETEROCYCLES. 24, No. 10, 2863-2870, 1986, and G. Montaudo and S. Caccamese: Journal of Organic Chemistry, 38, No. 4, 710-716, 1973) (for example, for melting point, found: 274 to 275°C, value in literature: 274 to 278°C). As a result, the compound prepared as sample 1 was identified as α-truxillic acid.

### Synthesis Example 2

p-Coumaric acid (1.0 g) was provided, and then dispersed in hexane in a test tube. The dispersion was then irradiated with light for one day to induce photodimerization. After the completion of the reaction, the reaction product was washed several times with ether to give 980 mg of 4,4'-dihydroxy-α-truxillic acid (sample 2).

The compound thus obtained was measured for physicochemical properties in terms of melting point, NMR and the like. The results were compared with values reported in literature (W.H. Morrison, III, R.D. Hartley, and D.S. Himmelsbach: Journal of Agricultural and Food Chemistry, 40, 768-771, 1992) (for example, for melting point, found: 300°C or above, value in literature: 340°C). As a result, the compound prepared as sample 2 was identified as 4,4'-dihydroxy-α-truxillic acid.

### Synthesis Example 3

p-Nitrophenol (6.0 g) was dissolved in 60 ml of tetrahydrofuran. Pyridine (1.9 g) was then added to the solution, and the temperature in the system was held at 0 to 5°C. Thus, a reaction solution A was prepared. Next, a solution of 6.8 g of cinnamic acid chloride in 2 ml of tetrahydrofuran was gradually added dropwise to the reaction solution A to allow a reaction to proceed. After the reaction for 2 hr, the reaction solution was poured into 800 ml of water. The resultant precipitate was collected by filtration, and was then dried. The dried precipitate (6 g) was then dispersed in hexane. The dispersion was irradiated with light for 10 hr to give bis(p-nitrophenyl) β-truxinate (sample 3). Sample 3 was then purified by recrystallization from methyl ethyl ketone.

The compound thus obtained was measured for physicochemical properties in terms of melting point, NMR and the like. The results were compared with values reported in literature (T. Nishikubo, E. Takahashi, T. Miyaji, and T. Iizawa: Bulletin of Chemical Society Japan, 58, 3399-3400, 1985) (for example, for melting point, found: 195 to 196°C, value in literature: 192 to 193°C). As a result, the compound prepared as sample 3 was identified as bis(p-nitrophenyl) β-truxinate.

### Synthesis Example 4

Sample 3 (690 mg) and 360 mg of potassium hydroxide were added to 7 ml of methanol. The mixture was refluxed for 3 hr, and was then adjusted to pH 3 by the addition of hydrochloric acid. The reaction solution was poured into water. The resultant precipitate was collected by filtration, and was then dried to give β-truxinic acid (sample 4). Sample 4 was then purified by recrystallization from acetic acid.

The compound thus obtained was measured for physicochemical properties in terms of melting point, NMR and the like. The results were compared with values reported in literature (T. Nishikubo, E. Takahashi, T. Miyaji, and T. Iizawa: Bulletin of Chemical Society Japan, 58, 3399-3400, 1985, and G. Montaudo, S. Caccamese: Journal of Organic Chemistry, 38, No. 4, 710-716, 1973) (for example, for melting point, found: 208 to 209°C, value in literature: 209 to 210°C). As a result, the compound prepared as sample 4 was identified as β-truxinic acid.

### Synthesis Example 5

To 435 mg of α-truxillic acid were added 2.47 g of thionyl chloride and one drop of dimethylformamide. The mixture was refluxed for 3 hr. Thionyl chloride remaining unreacted was then removed in vacuo to give α-truxillic acid chloride. Separately, p-nitrophenol (230 mg) was dissolved in 3 ml of tetrahydrofuran. Pyridine (0.5 g) was then added to the solution, and the temperature in the system was held at 0 to 5°C. A solution of 500 mg of α-truxillic acid chloride prepared above in 1 ml of tetrahydrofuran was gradually added dropwise to this solution to allow a reaction to proceed. After the reaction for 2 hr, the reaction solution was poured into 500 ml of water. The resultant precipitate was collected by filtration, and was then dried to give bis(p-nitrophenyl) α-truxillate (sample 5). Sample 5 was then purified by recrystallization from methyl ethyl ketone.

Sample 5 thus obtained was measured for physicochemical properties. The results were as follows.
Melting point: 230-231°C
Positive FAB-MS (m/z): 538[M+H]+
1H-NMR (CDCl3-d) δ: 8.08 (4H, dd, J = 1.98, 6.92 Hz), 7.43 (12H, m), 6.45 (4H, dd, J = 1.98, 6.92 H2), 4.75 (4H, m), 4.34 (4H, m)

From the above physicochemical properties, the compound as sample 5 was identified as bis(p-nitrophenyl) α-truxillate.

### Synthesis Example 6

4-Chlorocinnamic acid (500 mg) was provided, and then dispersed in hexane in a test tube. The dispersion was then irradiated with light for two days to induce photodimerization. After the completion of the reaction, the reaction solution was filtered, followed by drying to give 4,4'-dichloro-β-truxinic acid (sample 6). Sample 6 was then purified by recrystallization from acetic acid.

The compound thus obtained was measured for physicochemical properties in terms of melting point, NMR and the like. The results were compared with values reported in literature (M.D. Cohen, G.M.J. Schmidt, and F.I. Sonntag: Journal of Chemical Society, 2000-2013, 1964) (for example, for melting point, found: 159 to 160°C, value in literature: 160°C). As a result, the compound prepared as sample 6 was identified as 4,4'-dichloro-β-truxinic acid.

### Synthesis Example 7

A solution of 4 ml of trimethylsilyldiazomethane in 10 ml of ether was gradually added dropwise to a solution of 1.0 g of α-truxillic acid (sample 1) in 40 ml of methyl ethyl ketone. The mixture was allowed to stand for one hr, and the reaction solution was then concentrated. The concentrate was subjected to column chromatography on silica gel (No. 9385, 500 ml, manufactured by MERCK), wherein a mobile phase (chloroform : methanol = 50 : 1) was used for elution to perform fractionation. Thus, fractions 1 to 40 (each 10 ml) were obtained. Dimethyl α-truxillate (sample 7) (308 mg) was obtained from the fractions 24 to 38. Sample 7 was purified by recrystallization from chloroform.

The compound thus obtained was measured for physicochemical properties in terms of melting point, NMR and the like. The results were compared with values reported in literature (A. Baracchi, S. Chimichi, F.D. Sio, D. Donati, R. Nesi, and P. Sarti-Fantoni: HETEROCYCLES. 24, No. 10, 2863-2870, 1986) (for example, for melting point, found: 172 to 174°C, value in literature: 174°C). As a result, the compound prepared as sample 7 was identified as dimethyl α-truxillate.

### Synthesis Example 8

A solution of 4 ml of trimethylsilyldiazomethane in 10 ml of ether was gradually added dropwise to a solution of 1.0 g of 4',4"-dihydroxy-α-truxillic acid (sample 2) in 40 ml of methyl ethyl ketone and 20 ml of MeOH. The mixture was allowed to stand for one hr, and the reaction solution was then concentrated. The concentrate was subjected to column chromatography on silica gel (No. 9385, 500 ml, manufactured by MERCK), wherein mobile phases (hexane : ethyl acetate = 3 : 1, 2 : 1, 1 : 1, and 1 : 2, each 600 ml) were used for successive elution to perform fractionation. Thus, fractions 1 to 140 (each 15 ml) were obtained. Dimethyl 4,4'-dihydroxy-a-truxillate (sample 8) (999.1 mg) was obtained from fractions 71 to 135. Sample 8 was purified by recrystallization from chloroform.

The compound thus obtained was measured for physicochemical properties in terms of melting point, NMR and the like. The results were compared with values reported in literature (H. Koshino, S. Terada, T. Yoshihara, T. Shimanuki, T. Sato, and A. Tajimi: Phytochemistry. 27, No. 5, 1333-1338, 1988) (for example, for melting point, found: 178 to 181°C, value in literature: 174 to 177°C). As a result, the compound prepared as sample 8 was identified as dimethyl 4,4'-dihydroxy-α-truxillate.

Samples 1 to 8 synthesized above are specifically summarized as follows.

| Sample | X₁ and X₂ | Y₁, Y₂, Z₁, and Z₂ | R₁ and R₂ |
|---|---|---|---|
| 1 | -H | -H | -OH |
| 2 | -OH | -H | -OH |
| 3 | -H | -H | -OC₆H₄NO₂ |
| 4 | -H | -H | -OH |
| 5 | -H | -H | -OC₆H₄NO₂ |
| 6 | -Cl | -H | -OH |
| 7 | -H | -H | -OCH₃ |
| 8 | -OH | -H | -OCH₃ |

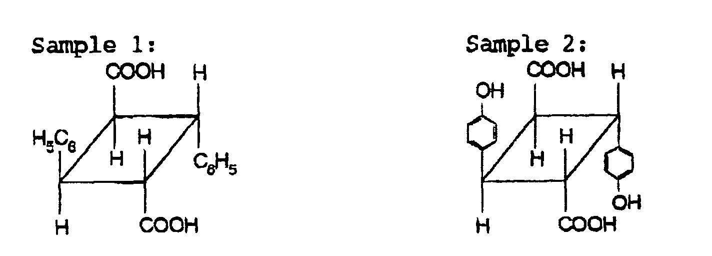

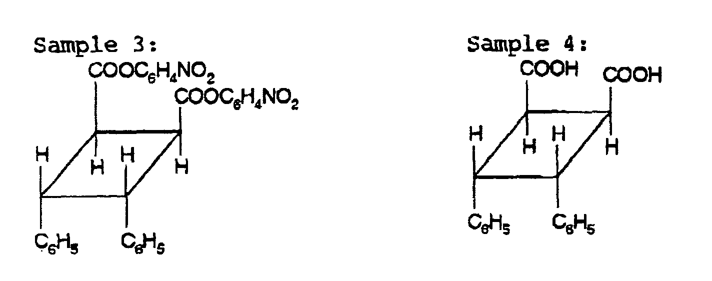

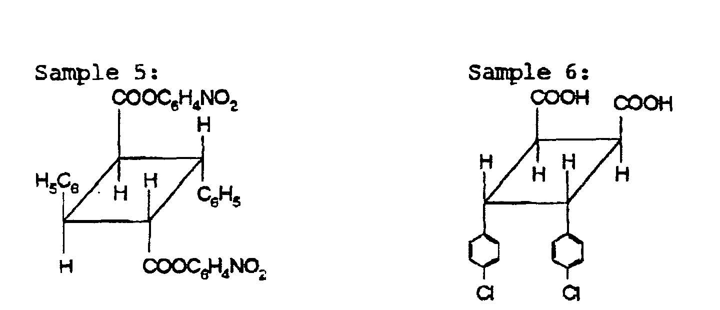

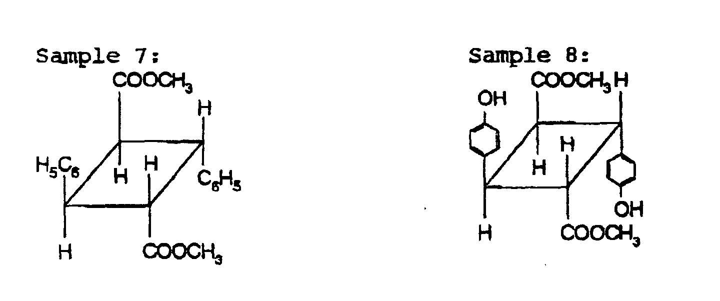

### Analgesic, anti-inflammatory activity test

An analgesic, anti-inflammatory activity test (the so-called "formalin test") was carried out using samples 1 to 9 as test drugs.

A 1.0% formalin solution (20 µl) was injected as a stimulant into the hypoderm of the planta depis in the hind leg of mice (five-week-old male ICR mice which, after purchase, had been subjected to quarantine and acclimation for one week). An act of licking the hind leg by the mice for alleviating the pain was then successively measured with a stopwatch 5 min by 5 min for 30 min. In this case, for each of the test drugs (samples 1 to 9), a suspension corresponding to a dose of 40 mg/kg was prepared using an aqueous solution of 0.5% polyoxyethylene sorbitan monooleate (Tween 80; a product of Atlas). The suspension (100 µl) for each sample was intraperitoneally administered to the mice 15 min before the stimulation. For a control group, the same volume of 0.5% Tween 80 was intraperitoneally administered to the mice.

It is generally known that the formalin test as described above causes a biphasic response, that is, the following first-phase response and second-phase response.

The first-phase response refers to a response indicated by pain in a 0 to 10 min period after the stimulation with formalin, that is, a response indicated by pain caused by direct stimulation of the sensory nerve at its end upon the injection of formalin. At the present time, only narcotic analgesics such as morphine are known to have inhibitory activity against this first-phase response. on the other hand, the second-phase response refers to a response indicated by pain in a 10 to 30 min period after the stimulation with formalin, that is, a response indicated by pain attributable to inflammation caused upon the injection of formalin. This response can be inhibited by conventional analgesics such as aspirin.

The analgesic, anti-inflammatory activity was evaluated in two separate phases, a phase from 0 min to 10 min after the administration and a phase from 10 min to 30 min after the administration. For each of the phases, the total number of seconds for which the mouse licked the hind leg (licking time) was calculated, and this value was compared with the value for the control.

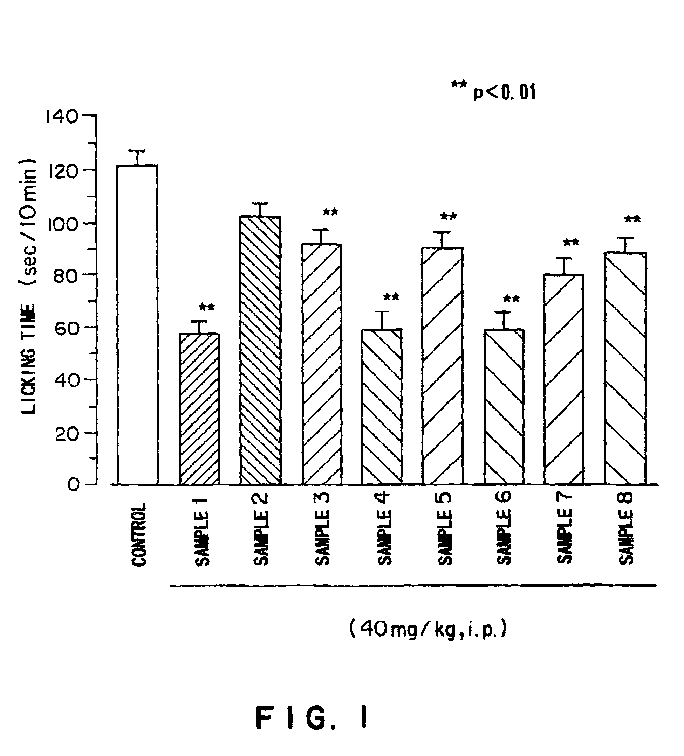

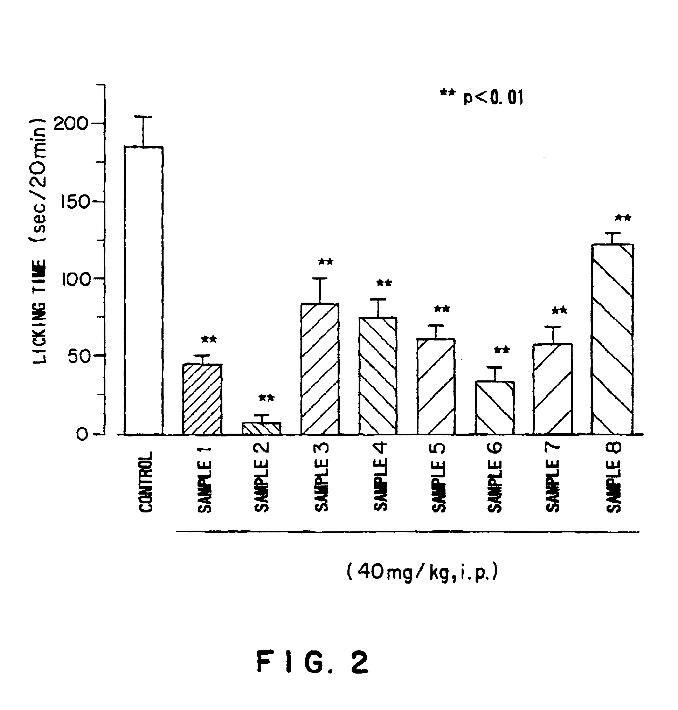

The results thus obtained (n = 10, average Value ± standard error) are shown in Table 1 below. Further, the results of the formalin test are shown in Figs. 1 and 2 which are respectively a graph showing the results of the formalin test for the first phase and a graph showing the results of the formalin test for the second phase.

**Table 1**

| Stimulation with 1.0% formalin (20 µl injected) sample: 40 mg/kg, intraperitoneal administration (10 mice per group) | | |
|---|---|---|
| | 1st phase response (0 to 10 min) | 2nd phase response (10 tc 30 min) |
| Control | 121.33 ± 5.32 | 183.51 ± 20.10 |
| Sample 1 | 57.56 ± 4.84 | 43.01 ± 4.83 |
| Sample 2 | 102.34 ± 4.09 | 7.12 ± 3.29 |
| Sample 3 | 92.18 ± 5.63 | 82.82 ± 18.47 |
| Sample 4 | 59.40 ± 7.19 | 74.75 ± 11.94 |
| Sample 5 | 90.63 ± 5.50 | 60.40 ± 10.45 |
| Sample 6 | 59.37 ± 6.04 | 34.44 ± 9.13 |
| Sample 7 | 80.81 ± 5.66 | 58.01 ± 12.26 |
| Sample 8 | 89.65 ± 4.67 | 122.70 ± 7.16 |
| Sample 9 | 86.97 ± 7.18 | 92.02 ± 18.41 |

### Acute gastric ulcer test

An acute gastric ulcer test was carried out using sample 2 and indometacin as test drugs. When indometacin was orally administered to rats at a dose of not less than 50 mg/kg, the occurrence of a gastric ulcer was clearly learned seven hr after the administration of indometacin. By contrast, for sample 2, the same test was carried out, except that, instead of indometacin, sample 2 was orally administered at a dose of 10 to 300 mg/kg. As a result, for all the cases, an ulcer did not occur at all.

## Claims

1. An analgesic agent comprising as an active ingredient a cyclobutanedicarboxylic acid derivative, containing substituted diphenyl, represented by formula (I): 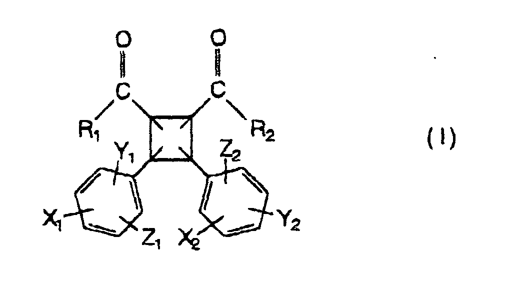 wherein X₁, X₂, Y₁, Y₂, Z₁, and Z₂, which may be the same or different, each independently represent a hydrogen atom, hydroxyl, a halogen atom, alkyl, alkoxy, or a nitrogen-containing group; and R₁ and R₂, which may be the same or different, each independently represent hydroxyl, a halogen atom, alkoxy, aryloxy, terpeneoxy, saccharide, or a nitrogen-containing group.

2. The analgesic agent according to claim 1, wherein, in formula (I), X₁ = X₂, Y₁ = Y₂, and Z₁ = Z₂.

3. The analgesic agent according to claim 1 or 2, wherein any one of X₁, Y₁ and Z₁ and any one of X₂, Y₂ and Z₂ both represent hydroxyl or a halogen atom while the remaining groups represent a hydrogen atom.

4. The analgesic agent according to any one of claims 1 to 3, wherein R₁ and R₂ each independently represent hydroxyl, methoxy, or nitrophenoxy.

5. A process for producing a cyclobutanedicarboxylic acid derivative for the analgesic agent according to any one of claims 1 to 4, said process comprising the steps of:
providing a cinnamic acid derivative represented by formula (II) 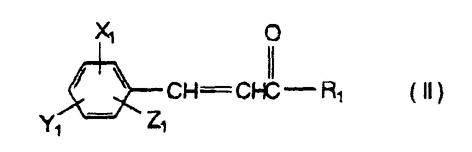 wherein X₁, Y₁, and Z₁, which may be the same or different, each independently represent a hydrogen atom, hydroxyl, a halogen atom, alkyl, alkoxy, or a nitrogen-containing group; and R₁ represents hydroxyl, a halogen atom, alkoxy, aryloxy, terpeneoxy, saccharide, or a nitrogen-containing group; and
dispersing the derivative in an organic solvent and then irradiating the dispersion with light to allow photodimerization to proceed.
